# EUROPÄISCHE PATENTSCHRIFT

(11) **EP 1 318 844 B1**
(45) Veröffentlichungstag und Bekanntmachung des Hinweises auf die Patenterteilung: **24.09.2014**
(21) Anmeldenummer: 01949179.4
(22) Anmeldetag: 24.07.2001
(51) Int. Cl.: A61L 27/28, A61L 27/06, A61L 27/50

(54) **OBERFLÄCHENMODIFIZIERTE IMPLANTATE**
SURFACE-MODIFIED IMPLANTS
IMPLANTS A SURFACE MODIFIEE

(30) Priorität: 26.07.2000 CH 148100
(43) Veröffentlichungstag der Anmeldung: 18.06.2003
(73) Patentinhaber: Straumann Holding AG, 4002 Basel (CH)
(72) Erfinder: SIMPSON, James, Percival, CH-4458 Eptingen (CH); STEINEMANN, Samuel, G., CH-1025 St. Sulpice (CH)
(74) Vertreter: Schaad, Balass, Menzl & Partner AG
(86) Internationale Anmeldenummer: PCT/CH2001/000456
(87) Internationale Veröffentlichungsnummer: WO 2002/007792

(56) Entgegenhaltungen:
- EP-A- 0 806 211
- WO-A-96/16611
- US-A- 5 258 098
- BUSER D ET AL.: "Influence of surface characteristics on bone integration of titanium implants. A histomorphometric study in miniature pigs" JOURNAL OF BIOMEDICAL MATERIALS RESEARCH, Bd. 25, Nr. 7, 1991, Seiten 889-901, XP008000118

## Beschreibung

Die vorliegende Erfindung betrifft oberflächenmodifizierte osteogene Implantate, welche zum Einsetzen in Knochen dienen und welche erheblich verbesserte Osteointegrationseigenschaften aufweisen, sowie Verfahren zu deren Herstellung.

Implantate, welche zum Einsetzen in Knochen dienen, wie beispielsweise Hüft- oder Kniegelenkprothesen oder in den Kiefer einzuschraubenden Stifte für den Aufbau künstlicher Zähne, sind an sich bekannt. Solche Implantate bestehen vorzugsweise aus Titan oder Titanbasislegierungen, wie z.B. Titan/Zirkonlegierungen, wobei diese zusätzlich Niob, Tantal oder andere gewebeverträgliche metallische Zusätze enthalten können. Zentrale Eigenschaften solcher Implantate sind die Stärke der Verankerung im Knochen sowie die Zeitspanne, in der die Integration erreicht wird. Osteointegration bedeutet demnach die kraftschlüssig solide und dauerhafte Verbindung zwischen Implantatoberfläche und Knochengewebe.

Wie fest das Implantat im Knochen verankert ist, kann mit mechanischen Messungen festgestellt werden, nämlich durch Messung der Kraft, sei es als Zug, Druck, Scherung oder Drehmoment, welche nötig sind, um das im Knochen verankerte Implantat aus seiner Verankerung herauszuziehen oder heraus zu drehen, d.h. einen Adhäsionsbruch zwischen der Oberfläche des Implantats und der mit dieser verbundenen Knochensubstanz herbeizuführen. Solche Messmethoden sind an sich bekannt und beispielsweise in Brunski, Clinical Materials, Vol. 10, 1992, pp. 153-201, beschrieben. Messungen haben gezeigt, dass sich Titan-Implantate mit glatter Oberflächenstruktur nur wenig im Knochen verankern, während Implantate mit aufgerauhter Oberfläche einen bezüglich der Zugfestigkeit merklich verbesserten Knochen-Implantat-Verbund ergeben.

In EP 0 388 575 wird deshalb vorgeschlagen, auf der Implantat-Oberfläche in einem ersten Schritt mittels Sandstrahlen eine Makrorauhigkeit aufzubringen und diese anschliessend mittels Behandlung in einem Säurebad mit einer Mikrorauhigkeit zu überlagern. So kann die Implantatoberfläche mittels Sandstrahlen aufgerauht und anschliessend mit einem Aetzmittel, z.B. Fluorwasserstoffsäure oder Chlorwasserstoffsäure/Schwefelsäuregemisch behandelt werden. Die so mit einer definierten Rauhigkeit versehene Oberfläche wird dann mit Lösungsmitteln und Wasser gereinigt und einer Sterilisationsbehandlung unterzogen.

Der chemische Zustand der Oberfläche von Titan und Titanbasislegierungen ist komplex. Es wird davon ausgegangen, dass die Oberfläche von Titanmetall in Luft und Wasser spontan oxydiert und dass dann an der Oberfläche, das heisst in der äussersten Atomschicht des Oxids, eine Reaktion mit Wasser stattfindet, wobei Hydroxylgruppen gebildet werden. Diese, Hydroxylgruppen enthaltende, Oberfläche wird in der Literatur als "hydroxylierte" Oberfläche bezeichnet. Siehe H.P. Boehm, Acidic and Basic Properties of Hydroxylated Metal Oxide Surfaces, Discussions Faraday Society, Vol. 52, 1971, pp. 264-275.

In Fresenius J. Anal. Chem. (1991) 341:412-415; Advances in Biomaterials, Vol. 9: Clinical Implant Materials (eds. G. Heimke, U. Soltész, A.J.C. Lee) Elsevier, Amsterdam (1990) 69-74 sowie in Schmid, Photoelektronen-Spektroskopie zur Adsorption von Aminosäuren auf oxidiertem Titan, Dissertation, Universität Lausanne, 1992 werden XPS Studien von Aminosäuren, die auf Titanoxidoberflächen adsorbiert werden, offenbart. Dazu wurde die Oberfläche von Glasplättchen mit einer Titanschicht von 400 bis 500°A beschichtet und die Adsorptionsfähigkeit von verschiedenen Aminosäuren bei unterschiedlichem pH getestet. Der Nachweis der Adsorbate erfolgte mittels Photoelektronen-Spektroskopie (XPS). Die pH-Werte, bei denen Adsorption stattfand, und die Signalintensitäten lassen Rückschlüsse auf die Adsorptionsreaktionen zu. Demnach bildet die Titanoberfläche bevorzugt einen Oberflächenkomplex mit der Carboxylgruppe der Aminosäure in saurer Lösung. Die daraus resultierenden Positionen der adsorbierten Moleküle konnten durch Auswertungen der Linienintensitäten bestätigt werden.

Es wurde nun gefunden, dass eine hydroxylierte Oberfläche von an der Oberfläche oxydiertem Titanmetall oder oxydierter Titanbasislegierung biologisch wirksame Eigenschaften aufweist, da der metallische Fremdkörper sich mit dem Knochengewebe kraftschlüssig verbindet, das heisst osteointegriert.

Es hat sich überraschenderweise gezeigt, dass eine solche hydroxylierte und biologisch wirksame Oberfläche ihre Wirksamkeit über eine längere Zeitspanne behält und erheblich schneller mit der Knochensubstanz zu einem starken Verbund zusammen wächst, als eine gleiche nicht erfindungsgemäss behandelte und üblicherweise an der Luft getrocknete Oberfläche, wenn diese hydroxylierte Oberfläche mit einer Verbindung, welche im Molekül mindestens zwei Gruppen enthält, welche unabhängig voneinander eine primäre Aminogruppe (-NH₂), eine sekundäre Aminogruppe (-NH-), eine Carboxylgruppe (-COOH), eine Amidgruppe (-C(O)NH-), eine Phosphonogruppe (-P(O)(OH)₂) und/oder Hydroxyl bedeuten, oder mit einem Gemisch solcher Verbindungen, behandelt wurde bzw. diese hydroxylierte Oberfläche mit einer solchen Verbindung oder einem Gemisch solcher Verbindungen mindestens teilweise belegt wurde. Derart wird ein osteogenes Implantat mit verbesserten Osteointegrationseigenschaften, insbesondere auch mit einer beschleunigten Verankerungsreaktion, erhalten, wobei die biologische Wirksamkeit der erfindungsgemäss behandelten hydroxylierten Implantatoberflächen weitgehend unverändert bis zum Einsetzen des Implantats erhalten bleibt.

Die vorliegende Erfindung ist in den Patentansprüchen definiert. Insbesondere betrifft die vorliegende Erfindung ein oberflächenmodifiziertes osteogenes Implantat mit verbesserten Osteointegrationseigenschaften bzw. mit verbesserter Osteointegration, wobei dieses Implantat aus Titanmetall oder einer Titanbasislegierung besteht und zumindest teilweise eine aufgerauhte Oberfläche aufweist, dadurch gekennzeichnet, dass diese Oberfläche im hydroxylierten Zustand mit einer Verbindung, welche im Molekül mindestens zwei Gruppen enthält, welche unabhängig voneinander eine primäre Aminogruppe, eine sekundäre Aminogruppe, eine Carboxylgruppe, eine Amidgruppe, eine Phosphonogruppe und/oder Hydroxyl bedeuten, oder mit einem Gemisch solcher Verbindungen, mindestens teilweise belegt wurde. Vorzugsweise hat diese Verbindung ein Molekulargewicht nicht höher als 2000.

Vorzugsweise wird diese Oberfläche in einer gas- und flüssigkeitsdichten Umhüllung und in einer gegenüber der Implantantoberfläche inerten Atmosphäre verschlossen aufbewahrt, das heisst, dass sich im Innern der Umhüllung keine Verbindungen befinden, welche die biologische Wirksamkeit der Implantatoberfläche beeinträchtigen können.

Vorzugsweise ist das Innere der Umhüllung mit gegenüber der Implantatoberfläche inerten Gasen, wie z.B. Sauerstoff, Stickstoff, Edelgase oder einem Gemisch solcher Gase, befüllt. Das Innere der Umhüllung kann aber auch mindestens teilweise mit reinem Wasser, welches gegebenenfalls Zusatzstoffe enthält, befüllt sein, wobei mindestens eine solche Menge Wasser anwesend ist, dass die Benetzung der aufgerauhten Implantatoberfläche gewährleistet ist. Das Restvolumen innerhalb der Umhüllung kann mit gegenüber der Implantatoberfläche inerten Gasen, wie z.B. Sauerstoff, Stickstoff, Edelgase oder einem Gemisch solcher Gase befüllt sein.

Vorzugsweise enthält das im Inneren der Umhüllung anwesende reine Wasser als Zusatzstoff bzw. Zusatzstoffe mindestens eine Verbindung, welche im Molekül mindestens zwei Gruppen enthält, welche unabhängig voneinander eine primäre Aminogruppe, eine sekundäre Aminogruppe, eine Carboxylgruppe, eine Amidgruppe, eine Phosphonogruppe und/oder Hydroxyl bedeuten, oder ein Gemisch solcher Verbindungen, das heisst mindestens eine Verbindung, welche erfindungsgemäss zur Behandlung und mindestens teilweiser Belegung der Implantatoberfläche verwendet werden kann.

Die vorliegende Erfindung betrifft auch Verfahren zur Herstellung der erfindungsgemässen Implantate sowie die erfindungsgemäss hergestellten Implantate.

Vorzugsweise bestehen die erfindungsgemässen Implantate aus einer Titanbasislegierung, vorzugsweise aus einer Titan/Zirkonlegierung, wobei diese zusätzlich Niob, Tantal oder andere gewebeverträgliche metallische Zusätze enthalten können. Diese Implantate dienen vorzugsweise als Hüft- oder Kniegelenkprothesen oder als in den Kiefer einzuschraubenden Stifte für den Aufbau künstlicher Zähne. Solche Implantate, deren Beschaffenheit und die für deren Herstellung verwendeten metallischen Materialien sind an sich bekannt und beispielsweise in J. Black, G. Hastings, Handbook of Biomaterials Properties, Seiten 135-200, Verlag Chapman & Hall, London, 1998, beschrieben.

Untersuchungen haben gezeigt, dass die genügende Verankerung eines Implantats im Knochen in hohem Mass von der Oberflächenbeschaffenheit des Implantats, insbesondere von der Rauhigkeit, abhängt. Gemäss der vorliegenden Erfindung wird die biologische Wirksamkeit der erfindungsgemäss behandelten Oberfläche zur im wesentlichen physikalischen Wirkung der Oberflächenrauhigkeit synergistisch hinzugefügt, woraus eine erhebliche Verbesserung der Osteointegration resultiert. Das erfindungsgemässe Zahnimplantat weist vorzugsweise eine Makrorauhigkeit, wie z.B. ein Schraubengewinde oder Vertiefungen in der Oberfläche auf, welche z.B. durch mechanische Bearbeitung und Strukturierung, Kugelstrahlen oder Sandstrahlen erhalten werden kann. Zusätzlich weist diese aufgerauhte Oberfläche vorzugsweise eine überlagerte Mikrorauhigkeit auf, wobei diese Mikrorauhigkeit vorzugsweise durch chemische Ätzung der Oberfläche oder mittels elektrochemischer (elektrolytischer) Behandlung oder durch eine Kombination dieser Verfahren hergestellt wird. Dabei erhält man eine hydroxylierte und gleichzeitig auch hydrophile Oberfläche. Diese hydroxylierte Oberfläche wird erfindungsgemäss mit einer Verbindung, welche im Molekül mindestens zwei Gruppen enthält, welche unabhängig voneinander eine primäre Aminogruppe, eine sekundäre Aminogruppe, eine Carboxylgruppe, eine Amidgruppe, eine Phosphonogruppe und/oder Hydroxyl bedeuten, oder mit einem Gemisch solcher Verbindungen, behandelt.

Die hydroxylierte Oberfläche kann beispielsweise hergestellt werden, indem man die Oberfläche mit der gewünschten Rauhigkeit bzw. Textur versieht, insbesondere indem man zuerst die Implantatoberfläche kugelstrahlt, sandstrahlt und/oder unter Verwendung von Plasmatechnik aufrauht, und anschliessend die mechanisch aufgerauhte Oberfläche mit einem elektrolytischen oder chemischen Prozess behandelt, bis eine hydroxylierte und hydrophile Oberfläche entstanden ist. Vorzugsweise ätzt man das Implantat mit einer anorganischen Säure oder einem Gemisch anorganischer Säuren, vorzugsweise mit Fluorwasserstoffsäure, Chlorwasserstoffsäure, Schwefelsäure, Salpetersäure oder einem Gemisch solcher Säuren oder aber die Oberfläche wird mit Chlorwasserstoffsäure, Wasserstoffperoxid und Wasser im Gewichtsverhältnis von etwa 1:1:5, aktiviert.

Vorzugsweise geht man so vor, dass man
- das Implantat kugelstrahlt und anschliessend mit verdünnter Fluorwassserstoffsäure bei Raumtemperatur ätzt und mit reinem destilliertem und CO₂-freiem Wasser wäscht; oder
- das Implantat sandstrahlt, z.B. mit Aluminiumoxid Partikeln mit einer durchschnittlichen Korngrösse von 0.1-0.25 mm oder 0.25-0.5 mm und anschliessend mit einem Chlorwasserstoffsäure/Schwefelsäuregemisch bei erhöhter Temperatur behandelt und mit reinem destilliertem und CO₂-freiem Wasser wäscht; oder
- das Implantat mit grobem Korn sandstrahlt, z.B. mit einem Korngemisch wie vorgängig definiert, und anschliessend mit einem Chlorwasserstoffsäure/Salpetersäuregemisch behandelt und mit reinem destilliertem und CO₂-freiem Wasser wäscht; oder
- das Implantat mit einem Gemisch von Chlorwasserstoff, Wasserstoffperoxid und Wasser im Gewichtsverhältnis von etwa 1:1:5 behandelt und mit reinem destilliertem und CO₂-freiem Wasser wäscht; oder
- das Implantat mittels der Verwendung von Plasmatechnik aufrauht und anschliessend in einem Gemisch von Chlorwasserstoff, Wasserstoffperoxid und Wasser im Gewichtsverhältnis von etwa 1:1:5 hydroxyliert und mit reinem destilliertem und CO₂-freiem Wasser wäscht; oder
- das Implantat in einem elektrolytischen Verfahren behandelt, wobei die Oberfläche gegebenenfalls vorgängig mechanisch aufgerauht wurde, und anschliessend mit reinem destilliertem und CO₂-freiem Wasser wäscht.

In allen Fällen wird das Implantat bzw. dessen hydroxylierte Oberfläche erfindungsgemäss direkt mit einer Verbindung, welche im Molekül mindestens zwei Gruppen enthält, welche unabhängig voneinander eine primäre Aminogruppe, eine sekundäre Aminogruppe, eine Carboxylgruppe, eine Amidgruppe, eine Phosphonogruppe und/oder Hydroxyl bedeuten, oder mit einem Gemisch solcher Verbindungen, behandelt. Insbesondere wird das Implantat bzw. dessen hydroxylierte Oberfläche nicht mit Alkohol, Aceton oder einem anderen organischen Lösungsmittel oder einem Desinfektionsmittel behandelt oder der Atmosphäre oder gasförmigen Substanzen, wie z.B. Kohlenwasserstoffen, ausgesetzt, welche gegenüber der hydroxylierten und hydrophilen Oberfläche nicht inert sind, und z.B. die hydrophile Oberflächeneigenschaft vermindern oder zerstören würden. Das im Verfahren verwendete "reine" Wasser enthält weder Kohlendioxid noch Dämpfe von Kohlenwasserstoffen sowie keine Alkohole, wie Methanol oder Ethanol, und kein Aceton oder verwandte Ketone. Es kann aber spezielle Zusatzstoffe enthalten, wie dies im weiteren beschrieben ist.

Das zum Waschen verwendete "reine" Wasser ist vorzugsweise mehrfach destilliertes oder via inverse Osmose hergestelltes Wasser, welches vorzugsweise in inerter Atmosphäre, das heisst z.B. unter erniedrigtem Druck, in Stickstoff- oder Edelgasatmosphäre hergestellt wurde. Insbesondere hat das reine Wasser eine elektrischen Widerstand von mindestens 2 Mohmcm (elektrischer Widerstand >2 Mohmcm) und einen Gesamtgehalt an organischem Kohlenstoff (total organic carbon, TOC) von höchstens 10 ppb (≤10 ppb).

Anschliessend an den Waschprozess wird das erhaltene Implantat vorzugsweise in reinem Wasser, welches gegebenenfalls Zusatzstoffe enthalten kann, aufbewahrt. Vorzugsweise wird das erhaltene Implantat in einer geschlossenen Umhüllung, welche mit einem gegenüber der Implantatoberfläche inerten Gas, beispielsweise Stickstoff, Sauerstoff oder Edelgas, wie z.B. Argon, befüllt ist und/oder in reinem Wasser, welches gegebenenfalls Zusatzstoffe enthält, bis zur weiteren erfindungsgemässen Bearbeitung aufbewahrt. Vorzugsweise ist die Umhüllung für Gase und Flüssigkeiten praktisch undurchlässig.

Erfindungsgemäss wird das Implantat, welches eine hydroxylierte Oberfläche aufweist bzw. die hydroxylierte Oberfläche des Implantats, im hydroxyliertem Zustand mit einer Verbindung, welche im Molekül mindestens zwei Gruppen enthält, welche unabhängig voneinander eine primäre Aminogruppe, eine sekundäre Aminogruppe, eine Carboxylgruppe, eine Amidgruppe, eine Phosphonogruppe und/oder Hydroxyl bedeuten, oder mit einem Gemisch solcher Verbindungen, behandelt und mit einer solchen Verbindung oder einem Gemisch solcher Verbindungen zumindest teilweise belegt. Diese Verbindungen können im weiteren auch eine oder mehrere Hydrosulfidgruppen (-SH-) enthalten. Solche Verbindungen müssen für den vorgesehenen pharmazeutischen Zweck pharmazeutisch zugelassen sein.

Für die Behandlung der hydroxylierten Implantatoberfläche sind Verbindungen bevorzugt, welche im Molekül mindestens zwei Gruppen enthalten, welche unabhängig voneinander eine primäre Aminogruppe, eine sekundäre Aminogruppe, eine Carboxylgruppe, eine Amidgruppe und/oder eine Phosphonogruppe bedeuten. Weiter bevorzugt sind Verbindungen, welche im Molekül mindestens zwei Gruppen enthalten, welche unabhängig voneinander eine primäre Aminogruppe, eine sekundäre Aminogruppe, eine Carboxylgruppe und/oder eine Amidgruppe bedeuten. Insbesondere bevorzugt sind Verbindungen, welche im Molekül mindestens zwei solche Gruppen enthalten, die voneinander verschieden sind. Bevorzugt sind Phosphoniumverbindungen, Aminosäuren und Polyaminosäuren, insbesondere Aminosäuren und Polyaminosäuren. Das Molekulargewicht dieser Verbindungen liegt, wie bereits erwähnt, vorzugsweise im Bereich bis zu 2000, vorzugsweise im Bereich von 60 bis 1500, vorzugsweise im Bereich von 200 bis 1100.

Verbindungen, welche mindestens eine primäre und/oder mindestens eine sekundäre Aminogruppe aufweisen sind beispielsweise Ethylendiamin, Trimethylendiamin, Verbindungen der Formel H₂N[(CH₂)₁₋₃NH]₁₋₄(CH₂)₁₋₃NH₂, wie H₂NCH₂CH₂NHCH₂CH₂NH₂, sowie verwandte oder homologe Verbindungen. Verbindungen, welche mindestens eine primäre und/oder mindestens eine sekundäre Aminogruppe sowie mindestens eine Hydroxylgruppe aufweisen sind beispielsweise Ethanolamin, Diethanolamin, Triethanolamin und verwandte oder homologe Verbindungen.

Verbindungen mit mindestens einer Carboxylgruppe und mindestens einer Hydroxylgruppe sind beispielsweise Hydroxycarbonsäuren mit 1-12 C-Atomen, wie Glykolsäure, beta-Hydroxy-propionsäure, beta-Hydroxy-buttersäure, gamma-Hydroxy-buttersäure oder 6-Hydroxycapronsäure.

Verbindungen, mit mindestens einer Carboxylgruppe und mindestens einer Aminogruppe sind beispielsweise die an sich bekannten Aminosäuren, wie Glycin, Alanin, Valin, Leucin, Phenylalanin, Tyrosin, Prolin, Hydroxyprolin, Serin, Threonin, Cystein, Cystin, Tryptophan, Asparaginsäure, Glutaminsäure, Arginin, Lysin, Histidin, sowie die weiteren an sich bekannten Aminosäuren. Weitere Beispiele sind gamma-Aminobuttersäure oder die Aminosalicylsäure.

Verbindungen mit mindestens einer Amidgruppe sind beispielsweise niedermolekulare Polyaminosäuren (Polypeptide), vorzugsweise niedermolekulare Polyaminosäuren, die aus beispielsweise 2 bis 10 Aminosäuren, vorzugsweise aus 3, 5 oder 7 Aminosäuren zusammengesetzt sind. Solche Polyaminosäuren sind in sehr grosser Zahl bekannt, wie beispielsweise Lys-Lys-Arg, Arg-Gly-Asp, Leu-Gly-Asp, Leu-Asp-Val, Gly-Arg-Gly-Asp-Ser, Gly-Arg-Gly-Asp-Tyr,
Val-Arg-Gly-Asp-Glu, Val-Arg-Gly-Asp-Phe, Arg-Glu-Asp-Arg-Val, Arg-Gly-Asp-Phe-Val, Arg-Gly-Asp-Phe-Lys, Arg-Gly-Asp-Ser-Lys, Arg-Ala-Asp-Phe-Val, Tyr-Ile-Gly-Ser-Asp, Ile-Lys-Val-Ala-Val, Arg-Glu-Asp-Arg-Val, Asp-Gly-Glu-Ala-Lys, Lys-Gln-Ala-Gly-Asp, Gly-Arg-Gly-Asp-Ser-Pro-Cys, Phe-His-Arg-Arg-Ile-Lys-Ala. Bevorzugt sind Polyaminosäuren, welche die Sequenzen Arg-Gly-Asp, oder Leu-Asp-Val, oder Arg-Glu-Asp-Arg-Val, oder Phe-His-Arg-Arg-Ile-Lys-Ala aufweisen. Als Polyaminosäuren kommen ebenso niedermolekulare Proteinfraktionen in Frage, wie solche beispielsweise bei der Herstellung von pflanzlicher oder tierischer Gelatine anfallen. Geeignet sind insbesondere solche Polypeptide, welche einen Mindestabstand zwischen Implantatoberfläche und der reaktiven Endgruppe von mindestens etwa 3.5 nm (Nanometer) aufweisen.

Geeignet sind auch Polyaminosäuren in cyklischer Form, beispielsweise cyclo(Arg-Gly-Asp-[D-Phenylalanin]-Lys), oder cyclo(Arg-Gly-Asp-[D-Valin]-Lys) oder cyclo[D-Val-Arg-Gly-Asp-Glu(-6Ahx-Tyr-Cys-NH₂-], welche beispielsweise mit einem linearen Peptid mit einer Ankergruppe verbunden sind. Die Polyaminosäuren können auch an andere Spacers gebunden sein, beispielsweise durch Reaktion mit epsilon-Aminohexansäure oder Polymere dieser Säure, beispielsweise dimere oder trimere Formen oder durch Reaktion mit 3-Merkaptobuttersäure, oder mit Ethylenglykol-Einheiten oder Diethylenglykoleinheiten. Als endständige Gruppen sind auch Reste der Formel (-NH-CH₂CH₂OCH₂CH₂OCH₂C(O)OH) und ähnliche Reste geeignet.

Geeignet sind auch Hydroxamsäuren der Formel R-C(O)NHOH, worin R [HO(CH₂CH₂O)₁₋₄(CH₂)₁₋₄]- bedeutet. Innerhalb der Bedeutung der vorliegenden Erfindung fallen Hydroxamsäuren unter Verbindungen, welche eine Amidgruppe (-C(O)NH-) aufweisen. Verbindungen mit einer Phosphonogruppe sind beispielsweise Verbindungen der Formel R₁-P(O) (OH)₂, worin R₁ [HO(CH₂CH₂O)₁₋₄ (CH₂)₁₋₄]- bedeutet. Beispiele solcher Verbindungen sind HOCH₂CH₂(OCH₂CH₂)₂OCH₂C(O)NHOH oder HOCH₂CH₂ (OCH₂CH₂)₂OCH₂P(O) (OH)₂.

Vorzugsweise haben die vorgehend genannten Verbindungen, welche im Molekül mindestens zwei Gruppen enthalten, welche unabhängig voneinander eine primäre Aminogruppe, eine sekundäre Aminogruppe, eine Carboxylgruppe, eine Amidgruppe, eine Phosphonogruppe und/oder Hydroxyl bedeuten, ein Molekulargewicht von nicht höher als 2000, vorzugsweise im Bereich von 60 bis 1500, und vorzugsweise im Bereich von 200 bis 1100.

Zahlreiche der oben genannten Verbindungen können als Verbindungen der allgemeinen Formel (I):

(A)ₚ (CₙH₂ₙ₊₂₋ₚ₋ᵣ) (B)r (I),

beschrieben werden, worin
- die einzelnen Substituenten A in demselben Molekül unabhängig voneinander Carboxyl, Phosphono, -C(O)NHOH, Phenyl, Hydroxyphenyl, 4-Imidazolyl, Guanidino und/oder 3-Indolyl; vorzugsweise Carboxyl, Phosphono, Phenyl, Hydroxyphenyl, 4-Imidazolyl, Guanidino und/oder 3-Indolyl;
- die einzelnen Substituenten B in demselben Molekül unabhängig voneinander Hydroxyl, Amino (-NH-/-NH₂), Amido (-C[O]NH-), Hydroxymethylen (-CH₂OH) und/oder Hydrosulfid (-SH-); vorzugsweise Hydroxyl, Amino und/oder Amido;

n eine ganze Zahl von 1 bis 12, vorzugsweise 1 bis 8, vorzugsweise 1, 2, 3 oder 4;
p Null, 1, 2 oder 3;
r Null, 1, 2 oder 3;
die Summe von [p+r] eine ganze Zahl von 2 bis 6, vorzugsweise von 2, 3 oder 4;
2n+2-p-r mindestens 1, vorzugsweise mindestens 2, bedeuten. Der Rest (CₙH₂ₙ₊₂₋ₚ₋ᵣ) ist vorzugsweise linear oder Isopropyl.

Geeignet sind auch Verbindungen der allgemeinen Formel (II) und der Formel (IIa):

(D-L) (OCH₂CH₂O) (CH₂CH₂O)ₘ (Q-G) (II),

(D-L) (OCH₂CH₂CH₂O) (CH₂CH₂CH₂O)ₘ(Q-G) (IIa),

worin
- die einzelnen Substituenten D in demselben Molekül unabhängig voneinander Carboxyl, Phosphono, oder -C(O)NHOH oder eine der Bedeutungen von G; vorzugsweise Carboxyl, Phosphono, oder
- C(O)NHOH; vorzugsweise Carboxyl oder Phosphono;
- die einzelnen Substituenten G in demselben Molekül unabhängig voneinander Wasserstoff, Amino (-NH₂), Amido (-C(O)NH₂), Hydroxymethylen (-CH₂OH), Hydrosulfid (-SH-) oder eine der Bedeutungen von D; vorzugsweise Wasserstoff, Amino, Amido, Hydroxymethylen, Hydrosulfid; vorzugsweise Wasserstoff, Amino, Amido;
- L und Q unabhängig voneinander die direkte Bindung, oder einen Linker zur Bindung der Substituenten D und/oder G, vorzugsweise -(CₙH₂ₙ)-, worin n eine ganze Zahl von 1 bis 8, vorzugsweise -CH₂-, oder -CH₂CH₂-;
- m Null, oder eine ganze Zahl von 1 bis 8, vorzugsweise Null, 1, 2, 3, 4 oder 5, vorzugsweise Null oder 1, vorzugsweise Null, bedeuten.

L und Q bedeuten die direkte Bindung vorzugsweise für G = Wasserstoff oder Hydroxymethylen. Für die übrigen Bedeutungen von D und G bedeuten L und Q vorzugsweise - (CₙH₂ₙ) -.

Beispiele für die Verbindungen der Formel (II) sind in den vorgehenden Abschnitten bereits genannt.

Bevorzugt sind Aminosäuren und Polyaminosäuren, insbesondere Polyaminosäuren, welche die Sequenz Arg-Gly-Asp aufweisen, wie Arg-Gly-Asp selbst, Gly-Arg-Gly-Asp-Ser oder Arg-Gly-Asp-Arg-Gly-Asp.

Methoden zur Charakterisierung und Analyse von Metalloberflächen sind an sich bekannt. Diese Methoden können auch für die Messung und Kontrolle bzw. Überwachung der Belegungsdichte verwendet werden. Derartige an sich bekannte Analysenmethoden sind beispielsweise Infrarot-Spektroskopie, Laser-Desorption-MassenSpektroskopie (LDMS), Röntgenstrahlen angeregte Photoelektronenspektroskopie (XPS), Matrix-Assisted-Laser-Desorption-Ion-Mass-Spektrospkopie (MALDI), Time-of-Flight-Sekundär-Ionen-MassenSpektroskopie (TOFSIMS), Elektronen und Ionen Mikroanalyse, Optical Waveguide Lightmode Spectroscopy (OWLS) oder X-Ray Photoelectron Diffraction (XPD) verwenden. Damit können beispielsweise die auf der Metalloberfläche verfügbaren Titanatome bzw. Hydroxylgruppen gemessen werden. In der Regel ergeben die auf der Metalloberfläche verfügbaren Metallatome bzw. Hydroxylgruppen die maximale Belegungsdichte der Oberfläche mit einer monomolekularen Schicht ("monolayer"). Mittels der angegebenen an sich bekannten Analysenmethoden kann die Konzentration und die Dicke der monomolekularen Schicht, welche insbesondere abhängig ist von der chemischen Zusammensetzung der Metalloberfläche, deren Vorbehandlung und der chemisorbierten Verbindung, gemessen werden. So weist beispielsweise Titanoxid etwa vier bis fünf reaktive, sauer oder basisch reagierende, Gruppen pro nm² Oberfläche auf. Dies bedeutet, dass die Oberfläche von Titanoxid mit etwa vier Molekülen einer Aminosäure oder Polyaminosäure pro nm² Oberfläche belegt werden kann. Erfindungsgemäss ist es bevorzugt, dass nur etwa 5% - 70%, bezogen auf die maximale Belegung der Metalloberfläche mit einer monomolekularen Schicht der angegebenen Verbindung belegt wird. Insbesondere bevorzugt ist erfindungsgemäss eine Belegung von 10% - 50% und insbesondere von etwa 20%, bezogen auf die maximale Belegung der Metalloberfläche mit einer monomolekularen Schicht. In diesem Sinne bleibt die Metalloberfläche durch die verbleibenden "freien" Hydroxylgruppen weiterhin zumindest teilweise hydroxyliert, so dass eine Kombination beider Effekte ein Implantat mit sehr guten Osteointegrationseigenschaften ergibt.

Die Verbindung, welche im Molekül mindestens zwei Gruppen enthält, welche unabhängig voneinander eine primäre Aminogruppe, eine sekundäre Aminogruppe, eine Carboxylgruppe, eine Amidgruppe, eine Phosphonogruppe und/oder Hydroxyl bedeuten, oder mit einem Gemisch solcher Verbindungen, bringt man auf die hydroxylierte Oberfläche des Implantats in einer geeigneten Methode auf, beispielsweise aus wässriger Lösung oder aus einem organischen Lösungsmittel oder auch mittels Besprühen mit der reinen Verbindung oder dem reinen Verbindungsgemisch. Die Verbindung wird so von der hydroxylierten Oberfläche adsorbiert und angebunden. Angebunden bedeutet hier, dass sie durch Spülen mit Wasser nicht ohne weiteres entfernt werden kann. Dabei genügt es, die Verbindung in wässeriger oder organischer Lösung in sehr geringer Konzentration, je nach Verbindung in einer Konzentration in der Grössenordnung von 0.01 µMol/l (Mikromol pro Liter) oder höher, zum Beispiel 0.01 µMol/l bis etwa 100 µMol/l, vorzugsweise 0.1 µMol/l bis etwa 10 µMol/l, vorzugsweise etwa 1 µMol/l, mit der hydroxylierten Metalloberfläche in Kontakt zu bringen, um die gewünschte Belegung zu herzustellen. Diese Konzentrationsgrenzen sind aber nicht kritisch. Die erreichte Belegungsdichte der Oberfläche mit den genannten Verbindungen wird insbesondere von deren Konzentration im flüssigen Träger, der Kontaktzeit und der Kontakttemperatur, und den verwendeten Säurewerten (pH-Werten) bestimmt.

In diesem Sinn betrifft die vorliegende Erfindung auch ein Verfahren zur Herstellung eines erfindungsgemässen Implantats, indem man die Implantatoberfläche kugelstrahlt, sandstrahlt und/oder unter Verwendung von Plasmatechnik aufrauht, dadurch gekennzeichnet, dass man anschliessend
(i) die mechanisch oder plasmatechnisch aufgerauhte Oberfläche mit einem elektrolytischen oder chemischen Ätzverfahren behandelt bis eine hydroxylierte Oberfläche entstanden ist, vorzugsweise mit einer anorganischen Säure oder einem Gemisch anorganischer Säuren, vorzugsweise mit Fluorwasserstoffsäure, Chlorwasserstoffsäure, Schwefelsäure, Salpetersäure, oder einem Gemisch solcher Säuren, oder Chlorwasserstoff, Wasserstoffperoxid und Wasser im Gewichtsverhältnis von etwa 1:1:5; und
(ii) die Oberfläche mit einer genannten Verbindung, welche im Molekül mindestens zwei Gruppen enthält, oder mit einem Gemisch solcher Verbindungen, mindestens teilweise belegt.

Die Belegung der hydroxylierten Metalloberfläche mit der genannten Verbindung, oder mit dem genannten Verbindungsgemisch, kann mit einer Chemisorption bzw. mit einer chemischen Anbindung erklärt werden. Das bedeutet, dass eine reaktive Gruppe der zugegebenen Verbindung mit der sich an der Metalloberfläche befindenden Hydroxylgruppe eine Kondensationsreaktion, beispielsweise gemäss der Formel:
≡TiOH + -CH₂C(O)OH → ≡TiOC (O) CH₂- + H₂O eingeht, wobei =Ti- ein Metallion an der Metalloberfläche bedeutet. Man kann der Oberfläche in Abhängigkeit des Säurewertes des die Oberfläche umgebenden Elektrolyten einen amphoteren Charakter zuschreiben, wobei eine Wechselwirkung zwischen der Säure im Elektrolyt und dem basisch reagierenden Hydroxyl auf der Oxidoberfläche bzw. dem Anion im Elektrolyt und dem sauer reagierenden Hydroxyl des Oxids besteht. Zur Erklärung der Oberflächenreaktionen können die Bildung von kovalenten Bindungen, elektrostatische Effekte und/oder die Bildung von Wasserstoffbrücken herangezogen werden. Die vorliegende Erfindung ist aber nicht an diese Erklärungen gebunden. Entscheidend ist die Tatsache, dass die hier beschriebene Oberflächenbehandlung die biologische Wirksamkeit der hydroxylierten Oberfläche bewahrt und verbessert.

Um die genannte Verbindung, welche im Molekül mindestens zwei Gruppen enthält, oder ein Gemisch dieser Verbindungen, an die Metalloberfläche anzubinden, geht man vorzugsweise so vor, dass man die Verbindung aus wässriger oder organischer Lösung, vorzugsweise aus wässriger Lösung, durch Benetzen, oder mittels Besprühen mit der reinen Verbindung, auf die Oberfläche aufbringt. Gegebenenfalls erhitzt man auf eine Temperatur von etwa 70°C bis 120°C, gegebenenfalls unter Druck. Ebenso kann mit UV-Strahlung die Anbindung der Verbindung an die Oberfläche gefördert werden. Eine weitere Methode besteht darin, dass man die Verbindung, je nach der Art der Verbindung, aus wässriger saurer oder basischer Lösung auf die Oberfläche aufbringt. Die Lösung weist in diesem Fall vorzugsweise einen Säurewert (pH-Wert) von zwischen 2 und 4 oder zwischen 8 und 11 auf. Anschliessend kann das Implantat gegebenenfalls auf eine Temperatur von etwa 70°C bis 120°C, gegebenenfalls unter Druck, erhitzt oder mit UV-Strahlung behandelt werden.

Vorzugsweise ist das erfindungsgemässe Implantat, mindestens jedoch dessen erfindungsgemäss belegte Oberfläche, in einer gas- und flüssigkeitsdichten Umhüllung verschlossen, wobei sich im Innern der Umhüllung keine Verbindungen befinden, welche die biologische Wirksamkeit der Implantatoberfläche beeinträchtigen können, das heisst, gegenüber der Implantatoberfläche inert sind. Diese gas- und flüssigkeitsdichte Umhüllung ist vorzugsweise eine verschweisste Ampulle aus Glas, Metall, einem synthetischen Polymeren oder einem anderen gas- und flüssigkeitsdichten Material oder einer Kombination dieser Materialien darstellt. Das Metall liegt vorzugsweise als dünne Metallfolie vor, wobei polymere Materialien und metallische Folien, aber auch Glas, in an sich bekannter Weise miteinander zu einer geeigneten Verpackung kombiniert werden können.

Vorzugsweise weist das Innere der Umhüllung eine inerte Atmosphäre auf und ist mit einem inerten Gas und/oder mindestens teilweise mit reinem Wasser, welches gegebenenfalls Zusatzstoffe enthält, befüllt. Ein geeigneter Zusatzstoff, welcher dem reinem Wasser erfindungsgemäss für die verbesserte Lagerung des Implantats zugesetzt werden kann, ist insbesondere eine Verbindung, welche im Molekül mindestens zwei Gruppen enthält, welche unabhängig voneinander eine primäre Aminogruppe, eine sekundäre Aminogruppe, eine Carboxylgruppe, eine Amidgruppe, eine Phosphonogruppe und/oder Hydroxyl bedeuten, oder mit einem Gemisch solcher Verbindungen, und insbesondere dieselbe Verbindung oder dasselbe Verbindungsgemisch, womit die Implantatoberfläche belegt worden ist. Dabei enthält das reine Wasser die genannte Verbindung oder das Verbindungsgemisch vorzugsweise in einer Konzentration im Bereich von etwa 0.01 µMol/l bis 100 µMol/l, vorzugsweise etwa 0.1 µMol/l bis 10 µMol/l und vorzugsweise in einer Konzentration von etwa 1 µMol/l.

Weitere geeignete Zusätze, welche dem reinem Wasser erfindungsgemäss zugesetzt werden können, sind einwertige Alkalikationen, wie Na⁺ oder K⁺, oder ein Gemisch von Na⁺ und K⁺, mit entsprechenden Anionen in Form anorganischer Salze, wie z.B. Natriumchlorid, Kaliumchlorid, Natrium- oder Kaliumchlorat, Natrium- oder Kaliumnitrat, Natrium- oder Kaliumphosphat oder ein Gemisch solcher Salze. Ebenso können auch zweiwertige Kationen in Form von wasserlöslichen anorganischen Salzen zugesetzt werden. Geeignete Kationen sind insbesondere Mg⁺², Ca⁺², Sr⁺² und/oder Mn⁺² in Form der Chloride, Chlorate, Nitrate oder deren Gemische. Geeignete anorganische Anionen sind auch Phosphat- und Phosphonatanionen, wobei darunter jeweils auch Monoorthophosphat-Anionen und Diorthophosphat-Anionen bzw. Monoorthophosphonat-Anionen und Diorthophosphonat-Anionen zu verstehen sind, in Kombination mit den genannten Kationen.

Bevorzugt sind solche anorganische Kationen und Anionen, welche bereits in der Körperflüssigkeit vorkommen, insbesondere in der jeweiligen physiologischen Konzentration und bei einem physiologischen Säurewert im Bereich von vorzugsweise 4 bis 9 und vorzugsweise bei einem Säurewert im Bereich von 6 bis 8. Bevorzugte Kationen sind Na⁺, K⁺, Mg^{**+**2} und Ca^{**+**2}. Das bevorzugte Anion ist Cl⁻. Bevorzugt liegt die Gesamtmenge der genannten Kationen bzw. Anionen jeweils im Bereich von etwa 50 mEq/l bis 250 mEq/l, vorzugsweise etwa 100 mEq/l bis 200 mEq/l und vorzugsweise bei etwa 150 mEq/l. Dabei bedeutet Eq/l (Formel-)Equivalentgewicht bzw. Eq/l entspricht dem Atomgewicht der Formeleinheit geteilt durch die Wertigkeit. mEq/l bedeutet Milliäquivalentgewicht pro Liter. Enthält die Umhüllung zweiwertige Kationen, insbesondere Mg^{**+**2}, Ca^{**+**2}, Sr^{**+**2} und/oder Mn⁺², alleine oder in Kombination mit den erwähnten einwertigen Kationen, so liegt die Gesamtmenge der anwesenden zweiwertigen Kationen vorzugsweise im Bereich von 1 mEq/l bis 20 mEq/l. Ebenso können die oben angegebenen organischen Verbindungen im Gemisch mit den angegebenen anorganischen Salzen gelöst im reinen Wasser vorliegen, wobei die angegebenen Konzentrationen für die anwesenden Zusätze weiterhin gelten und in der Regel genügen.

Methoden zur Messung der wirksamen Oberfläche eines metallischen Körpers sind an sich bekannt. So sind beispielsweise elektrochemische Messmethoden bekannt, welche ausführlich in P.W. Atkins, Physical Chemistry, Oxford University Press, 1994, beschrieben sind. Auch aus Rauhigkeitsmessungen kann die effektive Oberfläche als Quadrat des hybriden Parameters Lᵣ, d.h. dem Quadrat des Profillängenverhältnis erhalten werden. In der Norm DIN 4762 ist der Parameters Lᵣ definiert als das Verhältnis der Länge des gestreckten zweidimensionalen Profils und der vermessenen Distanz. Letztere Messung hat aber zur Voraussetzung, dass die vertikale und laterale Auflösung der Messmethode kleiner ist als 1µm und sogar nahe bei 0.1µm liegt.

Die Referenzfläche für alle diese Messmethoden ist die flache polierte Metalloberfläche. Die gemessenen Werte der aufgerauhten Oberfläche im Vergleich zu den an der flachen und polierten Oberfläche, geben an, um wieviel grösser die aufgerauhte Oberfläche ist, verglichen mit der flachen und polierten Oberfläche. In vitro Untersuchungen mit Knochenzellen und in vivo histomorphometrische Untersuchungen an erfindungsgemässen Implantaten weisen darauf hin, dass die osteogenen Eigenschaften der erfindungsgemässen Implantate besonders hoch sind, wenn die aufgerauhte Oberfläche vorzugsweise mindestens 1.5 mal und vorzugsweise mindestens zweimal so gross ist, wie die vergleichbare flache und polierte Oberfläche. Bevorzugt ist die aufgerauhte Implantatoberfläche mindestens 2 bis 12 mal so gross, und vorzugsweise etwa 2.5 bis 6 mal so gross, wie die vergleichbare flache und polierte Oberfläche.

Industriell hergestellte Oberflächen von Titan und Titanlegierungen für die Bearbeitung in Laboratorien und Kliniken weisen in der Regel Verunreinigungen auf, welche im wesentlichen aus Kohlenstoffverbindungen und Spuren von Stickstoff, Kalzium, Schwefel, Phosphor und Silizium bestehen. Diese Verunreinigungen konzentrieren sich in der äussersten Metalloxidschicht. Vorzugsweise enthält die hydroxylierte und hydrophile Implantatoberfläche höchstens 20 Atom-% Kohlenstoff, gemessen mit spektroskopischen Methoden, wie XPS oder AES oder andere an sich bekannten spektroskopischen Methoden.

Die folgenden Beispiele erläutern die Erfindung.

### Beispiel 1

A) Eine gängige Form eines Zahnimplantats in Form einer Schraube von 4 mm Durchmesser und 10 mm Länge wurde hergestellt. Die Rohform wurde zerspanend durch Drehen und Fräsen des zylindrischen Rohlings in an sich bekannter Weise erhalten. Die in den Knochen einzusetzende Oberfläche wurde nun gemäss EP 0 388 575 mit einer Makrorauhigkeit versehen, indem diese mit einem Korn der mittleren Korngrösse 0.25-0.50 mm sandgestrahlt wurde. Anschliessend wurde die aufgerauhte Oberfläche (Makrorauhigkeit) mit einem wässerigen Chlorwasserstoffsäure/Schwefelsäuregemisch mit einem Verhältnis von HCl:H₂SO₄:H₂O von 2:1:1 bei einer Temperatur von über 80°C während etwa fünf Minuten behandelt, so dass ein Verhältnis der aufgerauhten Implantatoberfläche zur vergleichbaren polierten Oberfläche von 3.6, gemessen mittels Voltametrie im wässerigen Elektrolyten mit 0.15M NaCl, (entsprechend einem Verhältnis von 3.9, gemessen mit Impedanzspektrometrie im 0.1 molaren Na₂SO₄-Elektrolyten), erhalten wurde. Das so geformte Implantat wurde mit reinem Wasser gewaschen.
B) Anschliessend wurde das im Abschnitt A) erhaltene Implantat in eine Lösung bestehend aus reinem Wasser, welches die Verbindung (HO)₂(O)P(CH₂)₄COOH (hergestellt in an sich bekannter Weise) in einer Konzentration von 100 µMol/l enthielt, in der sauren Lösung in einem Soxhlet-Apparat unter Stickstoff während zwei Stunden gekocht. Das Implantat wurde unter Stickstoff entnommen und mit reinem Wasser gewaschen. Messungen ergaben eine Belegung der Metalloberfläche von etwa 30%. Anschliessend wurde das Implantat
   a) direkt in einer Glasampulle, welche mit reinem Wasser gefüllt war, verschweisst, nach 4 Wochen geöffnet und implantiert;
   b) direkt in einer Glasampulle verschweisst, welche gefüllt war mit reinem Wasser, welches mit 0.2 M Natriumbikarbonat auf pH=9 eingestellt war, und das Pentapeptid Gly-Arg-Gly-Asp-Ser in einer Konzentration von 1 µMol/l enthielt. Die Glasampulle wurde nach 4 Wochen geöffnet, kurz in isotoner Kochsalzlösung gewaschen und implantiert;
   c) nach Abschluss der Behandlung gemäss Abschnitt A) in atmosphärischer Luft getrocknet und implantiert (Vergleichsversuch).

Die gemäss den Versuchen a), b) und c) erhaltenen Implantate wurden im Oberkiefer eines Minischweins implantiert. Die Verankerung im Knochen wurde als Lösedrehmoment der im Oberkiefer des Minischweins implantierten Schraube gemessen. Die erhaltenen Ergebnisse sind in Tabelle 1 angegeben.

**Tabelle 1**

| | Verankerung* nach 2 Wochen | Verankerung* nach 3 Wochen | Verankerung* nach 4 Wochen |
|---|---|---|---|
| | (Ncm) | (Ncm) | (Ncm) |
| Versuch a) | 30 | 70 | 120 |
| Versuch b) | 40 | 90 | 130 |
| Vergleichsversuch c) | 20 | 60 | 100 |

| | | | |
|---|---|---|---|
| * die Verankerung ist als Lösedrehmoment in Ncm (Durchschnittswerte) angegeben. | | | |

Die Ergebnisse gemäss den Versuchen a) und b) (erfindungsgemässe Implantate) zeigen, dass die entsprechenden Lösedrehmomente für die angegebenen Einheilzeiten deutlich über denjenigen von Versuch c) liegen. Diese zeigen kürzere Einheilzeiten und eine beschleunigte Osteointegration an.

### Beispiel 2

A) Die Oberfläche des Implantats wurde, wie in Beispiel 1, Abschnitt A) beschrieben, vorbereitet.
B) Anschliessend wird das im Abschnitt A) erhaltene Implantat in eine Lösung bestehend aus reinem Wasser, welches die Verbindung HOCH₂CH₂(OCH₂CH₂)₂OCH₂C(O)NHOH (hergestellt in an sich bekannter Weise) in einer Konzentration von 10 µMol/l enthielt, in der sauren Lösung in einem Soxhlet-Apparat unter Stickstoff während zwei Stunden gekocht. Das Implantat wurde unter Stickstoff entnommen und mit reinem Wasser gewaschen. Messungen ergaben eine Belegung der Metalloberfläche von etwa 20%. Anschliessend wurde das Implantat
d) direkt in einer Glasampulle, welche mit reinem Wasser gefüllt war, verschweisst, nach 4 Wochen geöffnet und implantiert;
e) direkt in einer Glasampulle verschweisst, welche gefüllt war mit reinem Wasser, welches mit 0.2 M Natriumbikarbonat auf pH=9 eingestellt war, und das cyklische Pentapeptid Asp-Ser-Lys-Arg-Gly in einer Konzentration von 0.1 bis 1 µMol/l enthielt. Die Glasampulle wurde nach 4 Wochen geöffnet, kurz in isotoner Kochsalzlösung gewaschen und implantiert;
f) nach Abschluss der Behandlung gemäss Abschnitt A) in atmosphärischer Luft getrocknet und implantiert (Vergleichsversuch).

Die gemäss den Versuchen d), e) und f) erhaltenen Implantate wurden im Oberkiefer eines Minischweins implantiert. Die Verankerung im Knochen wurde als Lösedrehmoment der im Oberkiefer des Minischweins implantierten Schraube gemessen. Die erhaltenen Ergebnisse stimmten praktisch mit den in der Tabelle 1 gegeben Werten überein.

### Beispiel 3

Beispiel 1 wurde wiederholt, jedoch mit der Massgabe, dass die Verbindung (HO)₂(O)P(CH₂)₄COOH in Abschnitt B) ersetzt wurde durch das Pentapeptid Gly-Arg-Gly-Asp-Ser. Analoge Resultate zu denjenigen der Tabelle 1 wurden erhalten.

### Beispiel 4

Beispiel 2 wurde wiederholt, jedoch mit der Massgabe, dass die Verbindung HOCH₂CH₂ (OCH₂CH₂)₂OCH₂C(O NHOH in Abschnitt B) ersetzt wurde durch das cyklische Pentapeptid Asp-Ser-Lys-Arg-Gly. Analoge Resultate zu denjenigen der Tabelle 1 wurden erhalten

### Beispiel 5

Die Beispiele 1 bis 4 [jeweils Abschnitte A) und B)] wurden wiederholt, jedoch mit der Massgabe, dass ein Implantat mit einem Verhältnis der aufgerauhten Implantatoberfläche zur vergleichbaren polierten Oberfläche von 1.9 (gemessen mit Impedanzspektrometrie im 0.1 molaren Na₂SO₄-Elektrolyten) hergestellt wurde. Hierzu wurde die Implantatoberfläche nur mechanisch, durch Drehen, geschnitten und anschliessend wie in Beispiel 1 angegeben geätzt. Das so erhaltene Implantat wurde mit reinem Wasser gewaschen. Anschliessend wurde das Implantat
g) direkt in einer Glasampulle verschweisst, welche gefüllt war mit reinem Wasser, welches mit 0.2 M Natriumbikarbonat auf pH=9 eingestellt war, und das Pentapeptid Gly-Arg-Gly-Asp-Ser in einer Konzentration von 0.1 bis 1 µMol/l enthielt. Die Glasampulle wurde nach 4 Wochen geöffnet, kurz in isotoner Kochsalzlösung gewaschen und implantiert;
h) mit atmosphärischer Luft getrocknet und implantiert (Vergleichsversuch).

Die gemäss den Versuchen g) und h) erhaltenen Implantate wurden im Oberkiefer eines Minischweins implantiert. Die Verankerung im Knochen wurde als Lösedrehmoment der im Oberkiefer des Minischweins implantierten Schraube gemessen. Die erhaltenen Ergebnisse sind in Tabelle 2 angegeben.

**Tabelle 2**

| | Verankerung* nach 2 Wochen | Verankerung* nach 3 Wochen | Verankerung* nach 4 Wochen |
|---|---|---|---|
| | (Ncm) | (Ncm) | (Ncm) |
| Versuch g) | 25 | 45 | 70 |
| Vergleichsversuch h) | 15 | 30 | 60 |

| | | | |
|---|---|---|---|
| * die Verankerung ist als Lösedrehmoment in Ncm (Durchschnittswerte) angegeben. | | | |

Die Ergebnisse gemäss Versuch g)(erfindungsgemässes Implantat) zeigen, dass die entsprechenden Lösedrehmomente für die angegebenen Einheilzeiten deutlich über denjenigen von Versuch h) liegen. Geht man davon aus, dass in der Zahnchirurgie für den Aufbau der Suprastruktur ein Lösedrehmoment von mindestens 35 Ncm als unbedingt nötig erachtet wird, so wird dieser Wert vom erfindungsgemässen Implantat spätestens nach drei Wochen erreicht.

### Beispiel 6

Analoge Versuche zu den Beispielen 1 und 2 werden durchgeführt, indem man die Verbindung (HO)₂(O)P(CH₂)₄COOH (aus Beispiel 1) und die Verbindung HOCH₂CH₂ (OCH₂CH₂)₂OCH₂C (O) NHOH (aus Beispiel 2) jeweils ersetzt wurden durch Glycin (Molekulargewicht [MW]: 75.07), Serin (MW:105.09) lys.lys.arg (MW: 466.58), Aminosalicylsäure, Ethylendiamin und Milchsäure. Dabei werden analoge Resultate zu den in der Tabelle 1 angegeben, erhalten.

## Patentansprüche

1. Osteogenes Implantat aus Titanmetall oder einer Titanbasislegierung, mit einer zumindest teilweise aufgerauhten Oberfläche, wobei diese Oberfläche im hydroxylierten Zustand mit einer Verbindung, welche im Molekül mindestens zwei Gruppen enthält, die unabhängig voneinander eine primäre Aminogruppe, eine sekundäre Aminogruppe, eine Carboxylgruppe, eine Amidgruppe, eine Phosphonogruppe und/oder Hydroxyl bedeuten, oder mit einem Gemisch solcher Verbindungen, mindestens teilweise belegt wurde, **dadurch gekennzeichnet, dass** die Metalloberfläche mit der Verbindung zu 5% bis 70%, bezogen auf die maximale Belegung der Metalloberfläche mit einer mono-molekularen Schicht belegt ist.

2. Implantat nach Anspruch 1, **dadurch gekennzeichnet, dass** die Oberfläche mit 10% bis 50% mit der Verbindung belegt ist.

3. Implantat nach Anspruch 2, **dadurch gekennzeichnet, dass** die Metalloberfläche mit der Verbindung zu etwa 20%, bezogen auf die maximale Belegung der Metalloberfläche mit einer molekularen Schicht, belegt ist.

4. Implantat nach einem der Ansprüche 1 bis 3, **dadurch gekennzeichnet, dass** die Metalloberfläche durch freie Hydroxylgruppen zumindest teilweise hydroxyliert ist.

5. Implantat nach einem der Ansprüche 1 bis 4, **dadurch gekennzeichnet, dass** dieses aus einer Titan/Zirkonlegierung, besteht, welche gegebenenfalls zusätzlich Niob, Tantal oder andere gewebeverträgliche metallische Zusätze enthält.

6. Implantat nach einem der Ansprüche 1 bis 5, **dadurch gekennzeichnet, dass** dieses eine Mikrorauhigkeit sowie eine der Mikrorauhigkeit überlagerte Mikrorauhigkeit aufweist, wobei die Mikrorauhigkeit durch chemische Ätzung der Oberfläche und/oder mittels elekcrolytischer Behandlung hergestellt wurde, vorzugsweise durch Ätzen mit einer anorganischen Säure oder einem Gemisch anorganischer Säuren, vorzugsweise mit Fluorwasserstoffsäure, Chlorwasserstoffsäure, Schwefelsaure, Salpetersäure oder einem Gemisch solcher Säuren oder durch Behandlung der Oberfläche mit Chlorwasserstoffsäure, Wasserstoffperoxid und Wasser im Gewichtsverhältnis von etwa 1:1:5.

7. Implantat nach einem der Ansprüche 1 bis 6, **dadurch gekennzeichnet, dass** die Verbindung, welche im Molekül mindestens zwei Gruppen enthält, ausgewählt ist aus der Gruppe der folgenden Verbindungen: Ethylendiamin, Trimethylendiamin; Verbindungen der Formel H₂N [(CH₂)₁₋₃NH]₁₋₄(CH₂) ₁₋₃NH₂, vorzugsweise H₂NCH₂CH₂NHCH₂CH₂NH₂, sowie verwandte oder homologe Verbindungen; Ethanolamin, Diethanolamin, Triethanolamin und verwandte oder homologe Verbindungen.

8. Implantat nach einem der Ansprüche 1 bis 6, **dadurch gekennzeichnet, dass** die Verbindung, welche im Molekül mindestens zwei Gruppen enthält., ausgewählt ist aus der Gruppe der folgenden Verbindungen: Hydroxycarbonsäuren mit 1-12 C-Atomen, vorzugsweise Glykolsäure, beta-Hydroxy-propionsäure, beta-Hydroxy-buttersäure, gamma-Hydroxy-buttersäure, 6-Hydroxy-capronsäure; an sich bekannte Aminosäuren, vorzugsweise Glycin, Alanin, Valin, Leucin, Phenylalanin, Tyrosin, Prolin, Hydroxyprolin. Serin, Threonin, Cystein, Cystein, Tryptophan, Asparaginsäure, Glutaminsäure, Arginin, Lysin, Histidin, gamma-Aminobuttersäure und/oder Aminosalicylsäure.

9. Implantat nach einem der Ansprüche 1 bis 6, **dadurch gekennzeichnet, dass** die Verbindung, welche im Molekül mindestens zwei Gruppen enthält, ausgewählt ist aus der Gruppe der niedermolekulare Polyaminosäuren, welche vorzugsweise aus 2 bis 10 Aminosäuren, vorzugsweise aus 3, 5 oder 7 Aminosäuren, zusammengesetzt sind, vorzugsweise Lys-Lys-Arg, Arg-Gly-Asp, Leu-Gly-ASp, Leu-Asp-Val, Gly-Arg-Gly-Asp-Ser, Gly-Arg-Gly-Asp-Tyr, Val-Arg-Gly-Asp-Glu, Val-Arg-Gly-Asp-Phe, Arg-Glu-Asp-Arg-Val, Arg-Gly-Asp-Phe-Val, Arg-Gly-Asp-Phe-Lys, Arg-Gly-Asp-Ser-Lys, Arg-Ala-Asp-Phe-Val, Tyr-Ile-Gly-Ser-Asp, Ile-Lys-Val-Ala-Val, Arg-Glu-Asp-Arg-Val, Asp-Gly-Glu-Ala-Lys, Lys-Gln-Ala-Gly-Asp, Gly-Arg-Gly-Asp-Ser-Pro-Cys, Phe-His-Arg-Arg-Ile-Lys-Ala.

10. Implantat nach Anspruch 9, **dadurch gekennzeichnet, dass** die Polyaminosäure die Sequenzen. Arg-Gly-Asp, oder Leu-Asp-Val, oder Arg-Glu-Asp-Arg-Val, oder Phe-His-Arg-Argr-Ile-Lys-Ala aufweist.

11. Implantat nach Anspruch 9, **dadurch gekennzeichnet, dass** die Polyaminosäure eine niedermolekulare Proteinfraktion darstellt, wie solche bei der Herstellung von pflanzlicher oder tierischer Gelatine anfallen.

12. Implantat nach Anspruch 9 oder 10, **dadurch gekennzeichnet, dass** die Polyaminosäure eine cyklische Polyaminosäure darstellt, vorzugsweise cyclo(Arg-Gly-Asp-[D-Phenylalanin]-Lys), oder cyclo(Arg-Gly-ABp-[D-Valin]-Lys) oder cyclo[D-Val-Arg-Gly-Asp-Glu (-Ahx-Tyr-Cys-NH₂-1, welche vorzugsweise mit einem linearen Peptid mit einer Ankergruppe verbunden ist, oder an einen Spacers gebunden ist, vorzugsweise durch Reaktion mit epsilon-Aminohexansäure oder einem Polymeren dieser Säure, vorzugsweise einer dimeren oder trimeren Form, oder durch Reaktion mit 3-Merkaptobuttersäure, oder als endständige Gruppe einen Rest der Formel (-NH-CH₃CH₂OCH₃CH₃OCH₂C (O) OH) aufweist.

13. Implantat nach einem der Ansprüche 9 bis 12, **dadurch gekennzeichnet, dass** die Polyaminosäure einen Mindestabstand zwischen Implantatoberfläche und der reaktiven Endgruppe von mindestens etwa 3.5 nm aufweist.

14. Implantat nach einem der Ansprüche 1 bis 6, **dadurch gekennzeichnet, dass** die Verbindung, welche im Molekul mindestens zwei Gruppen enthält, ausgewählt ist aus der Gruppe der folgenden Verbindungen: Hydroxycarbonsäuren mit 1-12 C-Atomen, vorzugsweise der Formel R-C(O)NHOH, worin R [HO(CH₂CH₂O)₁₋₄ (CH₂)₁₋₄]- bedeutet, vorzugsweise HOCH₂CH₂(OCH₂CH₂)₂OCH₂C (O) NHOH; Verbindungen mit einer Phoaphonogruppe, vorzugsweise der Formel R₁-P(O)(OH)₂, worin R₁ [HO(CH₂CH₂O)₁₋₄(CH₂)₁₋₄]-bedeutet, vorzugsweise HOCH₂CH₂ (OCH₂CH₂)₂OCH₂P(O)(OH)₂-

15. Implantat nach einem der Ansprüche 1 bis 13, **dadurch gekennzeichnet, dass** die Verbindung, welche im Molekül mindestens zwei Gruppen enthält, ein Molekulargewicht nicht höher als 2000, vorzugsweise im Bereich von 60 bis 1500, und vorzugsweise im Bereich von 200 bis 1100 aufweist.

16. Implantat nach einem der Ansprüche 1 bis 6, **dadurch gekennzeichnet, dass** die Verbindung, welche im Molekül mindestens zwei Gruppen enthält, eine Verbindung der allgemeinen Formel (I) :
(A)ₚ(CₙH₂ₙ₊₂₋ₚ₋r)(B)ᵣ (I),
darstellt, worin
- die einzelnen Substituenten A in demselben Molekül unabhängig voneinander Carboxyl, Phosphono, -C(O)NHOH, Phenyl, Hydroxyphenyl, 4-Imidazolyl, Guanidino und/oder 3-Indoxyl; vorzugsweise Carboxyl, Phosphono-, Phenyl, Hydroxyphenyl, 4-Imidazolyl, Guanidino und/oder 3-indoxyl;
- die einzelnen substitutenten B in demselben Molekül unabhängig voneinander Hydroxyl, Amino (-NH-/NH₂), Amido (-C[O]NH-), Hydroxymethylen (-CH₂OH) und/oder Hydrosulfid; (-SH-); vorzugsweise Hydroxyl, Amino und/oder Amido;
n eine ganze Zahl von 1 bis 12, vorzugsweise 1 bis 8, vorzugsweise 1, 2, 3 oder 4;
p Null, 1, 2 oder 3;
r Null, 1, 2 oder 3;
die Summe von [p+r] eine ganze Zahl von 2 bis 6, vorzugsweise von 2, 3 oder 4;
2n+2-p-r mindestens 1, vorzugsweise mindestens 2, bedeuten.

17. Implantat nach einem der Ansprüche 1 bis 6, **dadurch gekennzeichnet, dass** die Verbindung, welche im Molekül mindestens zwei Gruppen enthält, eine Verbindung der allgemeinen Formel (II) oder der Formel (IIa):
(D-L) (OCH₂CH₂O) (CH₂CH₂O)ₘ(Q-G) (II),
(D-L) (OCH₂CH₂CH₂O) (CH₂CH₂CH₂O)ₘ(Q-G) (IIa),
worin
- die einzelnen Substituenten D in demselben Molekül unabhängig voneinander Carboxyl, Phosphene, oder -C(O)NHOH oder eine der Bedeutungen von G; vorzugsweise Carboxyl, Phosphono, oder -C(O)NHOH; vorzugsweise Carboxyl oder Phosphano;
- die einzelnen Substituenten G in demselben Molekül unabhängig voneinander Wasserstoff, Amino (-NH₂), Amido (-C(O)NH₂), Hydroxymethylen (-CH₂OH), Hydrosulfid (-SH-) oder eine der Bedeutungen von D; vorzugsweise Wasserstoff, Amino, Amido, Hydroxymethylen, Hydrosulfid; vorzugsweise Wasserstoff, Amino, Amido;
- L und Q unabhängig voneinander die direkte Bindung, oder einen Linker zur Bindung der Substituenten D und/oder G, vorzugsweise -(CₙH₂ₙ)-, worin n eine ganze Zahl von 1 bis 8, vorzugsweise -CH₂-, oder -CH₃CH₂-;
- m Null oder eine ganze zahl von 1 bis 8, vorzugsweise Null, 1, 2, 3, 4 oder 5, vorzugsweise Null oder 1, vorzugsweise Null,
bedeuten.

18. Implantat nach einem der Ansprüche 1 bis 17, **dadurch gekennzeichnet, dass** dieses Implantat, mindestens jedoch dessen belegte Oberfläche, in einer gas- und flüssigkeitsdichten Umhüllung verschlossen ist, welche mit einem gegenüber der Implantatoberfläche inerten Gas, vorzugsweise mit Stickstoff, Sauerstoff oder einem Edelgas und/oder zumindest teilweise mit reinem Wasser, welches gegebenenfalls Zusatzstoffe enthält, befüllt ist.

19. Implantat nach Anspruch 18, **dadurch gekennzeichnet, dass** das reine wasser in der Umhüllung eine Verbindung, welche im Molekül mindestens zwei Gruppen enthält, welche unabhängig voneinander eine primäre Aminogruppe, eine sekundäre Aminogruppe, eine Carboxylgruppe, eine Amidgruppe, eine Phosphonogruppe und/oder Hydroxyl bedeuten, oder mit einem Gemisch solcher Verbindungen, enthält, vorzugsweise dieselbe Verbindung oder dasselbe Verbindungsgemisch, womit die Implantatoberfläche belegt ist.

20. Implantat nach Anspruch 19, **dadurch gekennzeichnet, dass** das reine Wasser die Verbindung, welche im Molekül mindestens zwei Gruppen enthält, oder das Verbindungsgemisch, in einer Konzentration im Bereich von 0.01 µMol/l bis 100 µMol/l, vorzugsweise 0.1 bis 10 µMol/l und vorzugsweise in einer Konzentration von etwa 1 µMol/l, enthält.

21. Implantat nach Anspruch 18, **dadurch gekennzeichnet, dass** das reine Wasser-anorganische Salze in Form von einwertigen Alkalikationen, vorzugsweise Na⁺ oder K⁺, oder ein Gemisch von Na⁺ und K⁺, mit entsprechenden Anionen und/oder zweiwertige Kationen in Form von wasserlöslichen anorganischen Salzen, vorzugsweise Mg⁺², Ca⁺², sr⁺² und/oder Mn⁺² in Form der Chloride, Chlorate, Nitrate, Phosphate und/oder Phosphonate, enthält,

22. Implantat nach Anspruch 18 oder 21, **dadurch gekennzeichnet, dass** das reine Wasser anorganische Salze in einer Gesamtmenge der genannten Kationen bzw. Anionen jeweils im Bereich von 50 mEq/l bis 250 mEq/l, vorzugsweise 100 mEq/l bis 200 mEq/l und vorzugsweise in einer Menge von etwa 150 mEq/l enthält.

23. Verfahren zur Herstellung eines Implantats nach einem der Ansprüche 1 bis 17, indem man die implantatoberfläche kugelstrahlt, sandstrahlt und/oder unter Verwendung von Plasmatechnik aufrauht, **dadurch gekennzeichnet, dass** man anschliessend
(i) die mechanisch oder plasmatechnisch aufgerauhten Oberflache mit einem elektrolytischen oder chemischen Ätzverfahren behandelt bis eine hydroxylierte Oberfläche entstanden ist, vorzugsweise mit einer anorganischen Säure oder einem Gemisch anorganischer Säuren, vorzugweise mit Fluorwasserstoffsäure, Chlorwasserstoffsäure, Schwefelsäure, Salpetersäure, oder einem Gemisch solcher sauren, oder Chlorwasserstoff, Wasserstoffperoxid und Wasser im Gewichtsverhältnis von etwa 1:1:5; und
(ii) die Oberfläche mit einer Verbindung, welche mindestens eine primäre und/oder sekundäre Aminogruppe, und/oder eine Carboxygruppe und/oder eine Amidgruppe und/oder Phosphonogruppe enthält., zu 5% bis 70%, bezogen auf die maximale Belegung der Metalloberfläche mit einer molekularen Schicht, belegt.

24. Verfahren nach Anspruch 23, **dadurch gekennzeichnet, dass** man die Verbindung, welche im Molekül mindestens zwei Gruppen enthält, welche unabhängig voneinander eine primäre Aminogruppe, eine sekundäre Aminogruppe, eine Carboxylgruppe, eine Amidgruppe, eine Phosphonogruppe und/oder Hydroxyl bedeuten, oder das Gemisch dieser Verbindungen, aus wässriger, gegebenenfalls saurer oder basischer, Lösung durch Benetzen, oder mittels Besprühen mit der reinen Verbindung, auf die Oberfläche aufbringt und anschliessend gegebenenfalls auf eine Temperatur von etwa 80°C bis 120°C, gegebenenfalls unter Druck, erhitzt.

25. Verfahren nach Anspruch 24, dadurch gekennzeichnat, dass man die Verbindung in wässeriger Lösung in einer Konzentration von mindestens 10 µMol/l (Mikromol pro Liter) mit der hydroxylierten Metalloberfläche in Kontakt zu bringt.

## Claims

1. Osteogenic implant made of titanium metal or a titanium-based alloy, having an at least partially roughened surface, wherein this surface in the hydroxylated state has been at least partially covered with a compound which comprises in the molecule at least two groups which are, independently of one another, a primary amino group, a secondary amino group, a carboxyl group, an amide group, a phosphono group and/or hydroxyl, or with a mixture of such compounds, **characterized in that**
the metal surface is from 5% to 70% covered with the compound, based on the maximum coverage of the metal surface with a mono-molecular layer.

2. The implant as claimed in claim 1, **characterized in that** the surface is from 10% to 50% covered with the compound.

3. The implant as claimed in claim 2, **characterized in that** the metal surface is about 20% covered with the compound, based on the maximum coverage of the metal surface with a molecular layer.

4. The implant according to any of claims 1 to 3, **characterized in that** the metal surface is at least partially hydroxylated by free hydroxyl groups.

5. The implant according to any of claims 1 to 4, **characterized in that** the latter consists of a titanium/zirconium alloy which optionally contains in addition niobium, tantalum or other tissue-compatible metallic additions.

6. The implant according to any of claims 1 to 5, **characterized in that** the latter has a macro-roughness, and a micro-roughness superimposed on the macro-roughness, where the micro-roughness has been produced by chemical etching of the surface and/or by means of electrolytic treatment, preferably by etching with an inorganic acid or a mixture of inorganic acids, preferably with hydrofluoric acid, hydrochloric acid, sulfuric acid, nitric acid or a mixture of such acids or by treatment of the surface with hydrochloric acid, hydrogen peroxide and water in a ratio of about 1:1:5 by weight.

7. The implant according to any of claims 1 to 6, **characterized in that** the compound which comprises in the molecule at least two groups is selected from the group of the following compounds: ethylenediamine, trimethylene-diamine; compounds of the formula H₂N[(CH₂)₁₋₃NH]₁₋₄(CH₂)₁₋₃NH₂, preferably H₂NCH₂CH₂NHCH₂CH₂NH₂, and related or homologous compounds; ethanolamine, diethanolamine, triethanolamine and related or homologous compounds.

8. The implant according to any of claims 1 to 6, **characterized in that** the compound which comprises in the molecule at least two groups is selected from the group of the following compounds: hydroxy carboxylic acids having 1-12 C atoms, preferably glycolic acid, beta-hydroxy-propionic acid, beta-hydroxybutyric acid, gamma-hydroxy-butyric acid, 6-hydroxycaproic acid; amino acids known per se, preferably glycine, alanine, valine, leucine, phenylalanine, tyrosine, proline, hydroxyproline, serine, threonine, cystein, cystine, tryptophan, aspartic acid, glutamic acid, arginine, lysine, histidine, gamma-amino-butyric acid and/or aminosalicylic acid.

9. The implant according to any of claims 1 to 6, **characterized in that** the compound which comprises in the molecule at least two groups is selected from the group of low molecular weight polyamino acids which are preferably composed of 2 to 10 amino acids, preferably of 3, 5 or 7 amino acids, preferably Lys-Lys-Arg, Arg-Gly-Asp, Leu-Gly-Asp, Leu-Asp-Val, Gly-Arg-Gly-Asp-Ser, Gly-Arg-Gly-Asp-Tyr, Val-Arg-Gly-Asp-Glu, Val-Arg-Gly-Asp-Phe, Arg-Glu-Asp-Arg-Val, Arg-Gly-Asp-Phe-Val, Arg-Gly-Asp-Phe-Lys, Arg-Gly-Asp-Ser-Lys, Arg-Ala-Asp-Phe-Val, Tyr-Ile-Gly-Ser-Asp, Ile-Lys-Val-Ala-Val, Arg-Glu-Asp-Arg-Val, Asp-Gly-Glu-Ala-Lys, Lys-Gln-Ala-Gly-Asp, Gly-Arg-Gly-Asp-Ser-Pro-Cys, Phe-His-Arg-Arg-Ile-Lys-Ala.

10. The implant according to claim 9, **characterized in that** the polyamino acid has the sequences Arg-Gly-Asp, or Leu-Asp-Val, or Arg-Glu-Asp-Arg-Val, or Phe-His-Arg-Arg-Ile-Lys-Ala.

11. The implant as claimed in claim 9, **characterized in that** the polyamino acid represents a low molecular weight protein fraction like those resulting in the production of vegetable or animal gelatin.

12. The implant as claimed in claim 9 or 10, **characterized in that** the polyamino acid is a cyclic polyamino acid, preferably cyclo(Arg-Gly-Asp-[D-phenylalanine]-Lys), or cyclo(Arg-Gly-Asp-[D-valine]-Lys) or cyclo[D-Val-Arg-Gly-Asp-Glu(-sAhx-Tyr-Cys-NH₂-], which is preferably connected to a linear peptide having an anchor group, or is linked to a spacer, preferably by reaction with epsilon-aminohexanoic acid or a polymer of this acid, preferably a dimeric or trimeric form, or by reaction with 3-mercaptobutyric acid, or has as terminal group a radical of the formula (-NH-CH₂CH₂OCH₂CH₂OCH₂C(O)OH).

13. The implant according to any of claims 9 to 12, **characterized in that** the polyamino acid has a minimum distance between implant surface and the reactive end group of at least about 3.5 nm.

14. The implant as claimed in any of claims 1 to 6, **characterized in that** the compound which comprises in the molecule at least two groups is selected from the group of the following compounds: hydroxy carboxylic acids having 1-12 C atoms, preferably of the formula R-C(O)NHOH, where R is [HO(CH₂CH₂O)₁₋₄(CH₂)₁₋₄]-, preferably HOCH₂CH₂(OCH₂CH₂)₂OCH₂C(O)NHOH; compounds having a phosphono group, preferably of the formula R₁-P(O)(OH)₂ in which R₁ is [HO(CH₂CH₂O)₁₋₄(CH₂)₁₋₄]-, preferably HOCH₂CH₂ (OCH₂CH₂)₂OCH₂P(O)(OH)₂.

15. The implant according to claims 1 to 13, **characterized in that** the compound which comprises in the molecule at least two groups has a molecular weight no greater than 2000, preferably in the range from 60 to 1500, and preferably in the range from 200 to 1100.

16. The implant according to claims 1 to 6, **characterized in that** the compound which comprises in the molecule at least two groups is a compound of the general formula (I):
(A)ₚ(CₙH₂ₙ₊₂₋ₚ₋ᵣ)(B)ᵣ (I)
in which
- the individual substituents A in the same molecule are, independently of one another, carboxyl, phosphono, -C(O) NHOH, phenyl, hydroxyphenyl, 4-imidazolyl, guanidino and/or 3-indolyl; preferably carboxyl, phosphono, phenyl, hydroxyphenyl, 4-imidazolyl, guanidino and/or 3-indolyl;
- the individual substituents B in the same molecule are, independently of one another, hydroxyl, amino (-NH-/NH₂), amido (-C[O]NH-), hydroxymethylene (-CH₂OH) and/or hydrosulfide (-SH-); preferably hydroxyl, amino and/or amido;
n is an integer from 1 to 12, preferably 1 to 8, preferably 1, 2, 3 or 4;
p is zero, 1, 2 or 3;
r is zero, 1, 2 or 3;
the total of [p+r] is an integer from 2 to 6, preferably from 2, 3 or 4;
2n+2-p-r is at least 1, preferably at least 2.

17. The implant as claimed in any of claims 1 to 6, **characterized in that** the compound which comprises in the molecule at least two groups, said compound being a compound of the general formula (II) or of the formula (IIa):
(D-L) (OCH₂CH₂O) (CH₂CH₂O)ₘ(Q-G) (II),
(D-L)(OCH₂CH₂CH₂O)(CH₂CH₂CH₂O)ₘ(Q-G) (IIa),
in which
the individual substituents D in the same molecule are, independently of one another, carboxyl, phosphono, or -C(O)NHOH or one of the meanings of G; preferably carboxyl, phosphono, or -C(O)NHOH; preferably carboxyl or phosphono;
the individual substituents G in the same molecule are, independently of one another, hydrogen, amino (-NH₂), amido (-C(O)NH₂), hydroxymethylene (-CH₂OH), hydrosulfide (-SH-) or one of the meanings of D; preferably hydrogen, amino, amido, hydroxymethylene, hydrosulfide; preferably hydrogen, amino or amido; L and Q are, independently of one another, the direct linkage, or a linker to link the substituents D and/or G, preferably -(CₙH₂ₙ)- in which n is an integer from 1 to 8, preferably -CH₂- or -CH₂CH₂-;
m is zero, or an integer from 1 to 8, preferably zero, 1, 2, 3, 4 or 5, preferably zero or 1, preferably zero.

18. The implant as claimed in any of claims 1 to 17, **characterized in that** this implant, but at least its covered surface, is enclosed in a gas- and liquid-tight envelope which is filled with a gas which is inert for the implant surface, preferably with nitrogen, oxygen or a noble gas and/or at least partially with pure water which optionally contains additives.

19. The implant as claimed in claim 18, **characterized in that** the pure water in the envelope contains a compound which comprises in the molecule at least two groups which are, independently of one another, a primary amino group, a secondary amino group, a carboxyl group, an amide group, a phosphono group and/or hydroxyl, or with a mixture of such compounds, preferably the same compound or the same mixture of compounds with which the implant surface is covered.

20. The implant as claimed in claim 19, **characterized in that** the pure water contains the compound which comprises in the molecule at least two groups, or the mixture of compounds, in a concentration in the range from 0.01 µmol/l to 100 µmol/l, preferably 0.1 µmol/l to 10 µmol/l and preferably in a concentration of about 1 µmol/l.

21. The implant as claimed in claim 18, **characterized in that** the pure water contains inorganic salts in the form of monovalent alkali metal cations, preferably Na⁺ or K⁺, or a mixture of Na⁺ and K⁺, with appropriate anions and/or divalent cations in the form of watersoluble inorganic salts, preferably Mg²⁺, Ca²⁺, Sr²⁺ and/or Mn²⁺ in the form of the chlorides, chlorates, nitrates, phosphates and/or phosphonates.

22. The implant according to claim 18 or 21, **characterized in that** the pure water contains inorganic salts in a total amount of said cations and anions in each case in the range from 50 mEq/l to 250 mEq/l, preferably 100 mEq/l to 200 mEq/l and preferably in an amount of about 150 mEq/l.

23. A process for producing an implant as claimed in any of claims 1 to 17, by the implant surface being shot peened, sandblasted and/or roughened by use of plasma technology, **characterized in that** subsequently
(i) the surface which has been roughened mechanically or by plasma technology is treated with an electrolytic or chemical etching process until a hydroxylated surface has been produced, preferably with an inorganic acid or a mixture of inorganic acids, preferably with hydrofluoric acid, hydrochloric acid, phosphoric acid, nitric acid, or a mixture of such acids, or hydrogen chloride, hydrogen peroxide and water in the ratio of about 1:1:5 by weight; and
(ii) the surface is from 5% to 70% covered with a compound which comprises at least one primary and/or secondary amino group and/or one carboxyl group and/or one amide group and/or phosphono group, based on the maximum coverage of the metal surface with a molecular layer.

24. A process as claimed in claim 23, **characterized in that** the compound which comprises in the molecule at least two groups which are, independently of one another, a primary amino group, a secondary amino group, a carboxyl group, an amide group, a phosphono group and/or hydroxyl, or the mixture of these compounds, is applied from aqueous, where appropriate acidic or basic, solution by wetting, or by spraying with the pure compound, to the surface, and subsequently heated where appropriate to a temperature of about 80°C to 120°C, where appropriate under pressure.

25. The process as claimed in claim 24, **characterized in that** the compound is brought into contact to with the hydroxylated metal surface in an aqueous solution in a concentration of at least 10 µmol/1 (micromole per liter).

## Revendications

1. Implant ostéogène en métal de titane ou en un alliage à base de titane présentant une surface rendue au moins partiellement rugueuse, cette surface étant revêtue au moins partiellement, à l'état hydroxylé, par un composé, qui contient dans la molécule au moins deux groupes, qui signifient, indépendamment l'un de l'autre, un groupe amino primaire, un groupe amino secondaire, un groupe carboxyle, un groupe amide, un groupe phosphono et/ou hydroxyle, ou par un mélange de ces composés, **caractérisé en ce que** la surface métallique est revêtue par le composé à raison de 5% à 70%, par rapport au revêtement maximal de la surface métallique par une couche monomoléculaire.

2. Implant selon la revendication 1, **caractérisé en ce que** la surface est revêtue à raison de 10% à 50% par le composé.

3. Implant selon la revendication 2, **caractérisé en ce que** la surface métallique est revêtue par le composé à raison d'environ 20%, par rapport au revêtement maximal de la surface métallique par une couche moléculaire.

4. Implant selon l'une quelconque des revendications 1 à 3, **caractérisé en ce que** la surface métallique est au moins partiellement hydroxylée par des groupes hydroxyle libres.

5. Implant selon l'une quelconque des revendications 1 à 4, **caractérisé en ce que** celui-ci est constitué par un alliage titane/zirconium, qui contient le cas échéant en plus du niobium, du tantale ou d'autres additifs métalliques compatibles avec les tissus.

6. Implant selon l'une quelconque des revendications 1 à 5, **caractérisé en ce qu'**il présente une macrorugosité ainsi qu'une microrugosité superposée à la macrorugosité, la microrugosité ayant été produite par attaque chimique de la surface et/ou par traitement électrolytique, de préférence par attaque par un acide inorganique ou par un mélange d'acides inorganiques, de préférence par de l'acide fluorhydrique, de l'acide chlorhydrique, de l'acide sulfurique, de l'acide nitrique ou un mélange de ces acides ou par traitement de la surface par de l'acide chlorhydrique, du peroxyde d'hydrogène et de l'eau dans un rapport pondéral d'environ 1:1:5.

7. Implant selon l'une quelconque des revendications 1 à 6, **caractérisé en ce que** le composé, qui contient dans la molécule au moins deux groupes, est choisi dans le groupe des composés suivants : éthylènediamine, triméthylènediamine ; les composés de formule H₂N[(CH₂)₁₋₃NH]₁₋₄(CH₂)₁₋₃NH₂, de préférence H₂NCH₂CH₂NHCH₂CH₂NH₂, ainsi que les composés apparentés ou homologues ; l'éthanolamine, la diéthanolamine, la triéthanolamine et les composés apparentés ou homologues.

8. Implant selon l'une quelconque des revendications 1 à 6, **caractérisé en ce que** le composé, qui contient dans la molécule au moins deux groupes, est choisi dans le groupe des composés suivants : les acides hydroxycarboxyliques comprenant 1-12 atomes de carbone, de préférence l'acide glycolique, l'acide bêta-hydroxypropionique, l'acide bêta-hydroxybutyrique, l'acide gamma-hydroxybutyrique, l'acide 6-hydroxycaproïque ; les acides aminés connus en soi, de préférence la glycine, l'alanine, la valine, la leucine, la phénylalanine, la tyrosine, la proline, l'hydroxyproline, la sérine, la thréonine, la cystéine, la cystine, le tryptophane, l'acide aspartique, l'acide glutamique, l'arginine, la lysine, l'histidine, l'acide gamma-aminobutyrique et/ou l'acide aminosalicylique.

9. Implant selon l'une quelconque des revendications 1 à 6, **caractérisé en ce que** le composé, qui contient dans la molécule au moins deux groupes, est choisi dans le groupe des poly(acides aminés) de bas poids moléculaire, qui sont de préférence composés de 2 à 10 acides aminés, de préférence de 3, 5 ou 7 acides aminés, de préférence Lys-Lys-Arg, Arg-Gly-Asp, Leu-Gly-Asp, Leu-Asp-Val, Gly-Arg-Gly-Asp-Ser, Gly-Arg-Gly-Asp-Tyr, Val-Arg-Gly-Asp-Glu, Val-Arg-Gly-Asp-Phe, Arg-Glu-Asp-Arg-Val, Arg-Gly-Asp-Phe-Val, Arg-Gly-Asp-Phe-Lys, Arg-Gly-Asp-Ser-Lys, Arg-Ala-Asp-Phe-Val, Tyr-Ile-Gly-Ser-Asp, Ile-Lys-Val-Ala-Val, Arg-Glu-Asp-Arg-Val, Asp-Gly-Glu-Ala-Lys, Lys-Gln-Ala-Gly-Asp, Gly-Arg-Gly-Asp-Ser-Pro-Cys, Phe-His-Arg-Arg-Ile-Lys-Ala.

10. Implant selon la revendication 9, **caractérisé en ce que** le poly(acide aminé) présente les séquences Arg-Gly-Asp ou Leu-Asp-Val ou Arg-Glu-Asp-Arg-Val ou Phe-His-Arg-Arg-Ile-Lys-Ala.

11. Implant selon la revendication 9, **caractérisé en ce que** le poly(acide aminé) représente une fraction protéinique de bas poids moléculaire, telle que celles qui sont obtenues lors de la préparation de gélatines végétales ou animales.

12. Implant selon la revendication 9 ou 10, **caractérisé en ce que** le poly(acide aminé) représente un poly(acide aminé) cyclique, de préférence cyclo(Arg-Gly-Asp-[D-phénylalanine]-Lys) ou cyclo(Arg-Gly-Asp-[D-valine]-Lys) ou cyclo[D-Val-Arg-Gly-Asp-Glu(-εAhx-Tyr-Cys-NH₂-], qui est de préférence lié à un peptide linéaire par un groupe d'ancrage ou à un écarteur, de préférence par réaction avec de l'acide epsilon-aminohexanoïque ou un polymère de cet acide, de préférence sous forme dimère ou trimère, ou par réaction avec de l'acide 3-mercaptobutyrique, ou présente, comme groupe terminal, un radical de formule (-NH-CH₂CH₂OCH₂CH₂OCH₂C(o)OH).

13. Implant selon l'une quelconque des revendications 9 à 12, **caractérisé en ce que** le poly(acide aminé) présente une distance minimale entre la surface de l'implant et le groupe terminal réactif d'au moins environ 3,5 nm.

14. Implant selon l'une quelconque des revendications 1 à 6, **caractérisé en ce que** le composé, qui contient dans la molécule au moins deux groupes, est choisi dans le groupe des composés suivants : les acides hydroxycarboxyliques comprenant 1-12 atomes de carbone, de préférence de formule R-C(O)NHOH, où R signifie [HO(CH₂CH₂O)₁₋₄(CH₂)₁₋₄]-, de préférence HOCH₂CH₂(OCH₂CH₂₎₂OCH₂C(O)NHOH ; les composés présentant un groupe phosphono, de préférence de formule R₁-P(O)(OH)₂, où R₁ signifie [HO(CH₂CH₂O)₁₋₄(CH₂)₁₋₄]-, de préférence HOCH₂CH₂(OCH₂CH₂) ₂OCH₂P(O)(OH)₂.

15. Implant selon l'une quelconque des revendications 1 à 13, **caractérisé en ce que** le composé, qui contient dans la molécule au moins deux groupes, présente un poids moléculaire qui n'est pas supérieur à 2000, de préférence dans la plage de 60 à 1500 et de préférence dans la plage de 200 à 1100.

16. Implant selon l'une quelconque des revendications 1 à 6, **caractérisé en ce que** le composé, qui contient dans la molécule au moins deux groupes, représente un composé de formule générale (I) :
(A)ₚ(CₙH₂ₙ₊₂₋ₚ₋ᵣ)(B)ᵣ (I),
dans laquelle
- les différents substituants A dans la même molécule signifient, indépendamment les uns des autres, carboxyle, phosphono, -C(O)NHOH, phényle, hydroxyphényle, 4-imidazolyle, guanidino et/ou 3-indolyle ; de préférence carboxyle, phosphono, phényle, hydroxyphényle, 4-imidazolyle, guanidino et/ou 3-indolyle ;
- les différents substituants B dans la même molécule signifient, indépendamment les uns des autres, hydroxyle, amino (-NH-/NH₂), amido (-C[O]NH-), hydroxyméthylène (-CH₂OH) et/ou hydrosulfure (-SH-); de préférence hydroxyle, amino et/ou amido ;
n signifie un nombre entier de 1 à 12, de préférence de 1 à 8, de préférence 1, 2, 3 ou 4 ;
p vaut zéro, 1, 2 ou 3 ;
r vaut zéro, 1, 2 ou 3 ;
la somme de [p+r] vaut un nombre entier de 2 à 6, de préférence 2, 3 ou 4 ;
2n+2-p-r vaut au moins 1, de préférence au moins 2.

17. Implant selon l'une quelconque des revendications 1 à 6, **caractérisé en ce que** le composé, qui contient dans la molécule au moins deux groupes, représente un composé de formule générale (II) ou de formule (IIa) :
(D-L)(OCH₂CH₂O)(CH₂CH₂O)ₘ(Q-G) (II),
(D-L)(OCH₂CH₂CH₂O)(CH₂CH₂CH₂O)ₘ(Q-G) (IIa),
où
- les différents substituants D dans la même molécule signifient, indépendamment les uns des autres, carboxyle, phosphono ou -C(O)NHOH ou une des significations de G ; de préférence carboxyle, phosphono ou -C(O)NHOH ; de préférence carboxyle ou phosphono ;
- les différents substituants G dans la même molécule signifient, indépendamment les uns des autres, hydrogène, amino (-NH₂), amido (-C(O)NH₂), hydroxyméthylène (-CH₂OH), hydrosulfure (-SH-) ou une des significations de D ; de préférence hydrogène, amino, amido, hydroxyméthylène, hydrosulfure ; de préférence hydrogène, amino, amido ;
- L et Q représentent, indépendamment les uns des autres, la liaison directe ou un lieur pour la liaison des substituants D et/ou G, de préférence -(CₙH₂ₙ)-, où n vaut un nombre entier de 1 à 8, de préférence -CH₂- ou -CH₂CH₂- ;
- m vaut zéro ou un nombre entier de 1 à 8, de préférence zéro, 1, 2, 3, 4 ou 5, de préférence zéro ou 1, de préférence zéro.

18. Implant selon l'une quelconque des revendications 1 à 17, **caractérisé en ce que** cet implant, au moins cependant sa surface revêtue, est enfermé dans une enveloppe étanche aux gaz et aux liquides, qui est remplie par un gaz inerte par rapport à la surface de l'implant, de préférence par de l'azote, de l'oxygène ou un gaz noble et/ou au moins partiellement par de l'eau pure, qui contient le cas échéant des additifs.

19. Implant selon la revendication 18, **caractérisé en ce que** l'eau pure dans l'enveloppe contient un composé, qui contient dans la molécule au moins deux groupes qui signifient, indépendamment l'un de l'autre, un groupe amino primaire, un groupe amino secondaire, un groupe carboxyle, un groupe amide, un groupe phosphono et/ou un groupe hydroxyle, ou un mélange de ces composés, de préférence le même composé ou le même mélange de composés que celui par lequel la surface d'implant est revêtue.

20. Implant selon la revendication 19, **caractérisé en ce que** l'eau pure contient le composé, qui contient dans la molécule au moins deux groupes, ou le mélange de composés, en une concentration dans la plage de 0,01 µmole/l à 100 µmoles/l, de préférence 0,1 µmole/l à 10 µmoles/l et de préférence en une concentration d'environ 1 µmole/l.

21. Implant selon la revendication 18, **caractérisé en ce que** l'eau pure contient des sels inorganiques sous forme de cations de métal alcalin monovalents, de préférence Na⁺ ou K⁺, ou un mélange de Na⁺ et K⁺, avec des anions correspondants et/ou de cations divalents sous forme de sels inorganiques solubles dans l'eau, de préférence Mg⁺², Ca⁺², Sr⁺² et/ou Mn⁺² sous forme de chlorures, de chlorates, de nitrates, de phosphates et/ou de phosphonates.

22. Implant selon la revendication 18 ou 21, **caractérisé en ce que** l'eau pure contient des sels inorganiques en une quantité totale des cations ou des anions mentionnés à chaque fois dans la plage de 50 méquiv/l à 250 méquiv/l, de préférence 100 méquiv/l à 200 méquiv/l et de préférence en une quantité d'environ 150 méquiv/l.

23. Procédé pour la réalisation d'un implant selon l'une quelconque des revendications 1 à 17, en ce que la surface de l'implant est grenaillée, sablée et/ou rendue rugueuse par une technique de plasma, **caractérisé en ce qu'**ensuite
(i) la surface rendue rugueuse par voie mécanique ou par technique de plasma est traitée par un procédé d'attaque électrolytique ou chimique jusqu'à la formation d'une surface hydroxylée, de préférence par un acide inorganique ou un mélange d'acides inorganiques, de préférence l'acide fluorhydrique, l'acide chlorhydrique, l'acide sulfurique, l'acide nitrique ou un mélange de ces acides, ou de l'acide chlorhydrique, du peroxyde d'hydrogène et de l'eau dans un rapport pondéral d'environ 1:1:5 ; et
(ii) la surface est revêtue par un composé, qui contient au moins un groupe amino primaire et/ou secondaire et/ou un groupe carboxyle et/ou un groupe amide et/ou un groupe phosphono, à raison de 5% à 70%, par rapport au revêtement maximal de la surface métallique par une couche moléculaire.

24. Procédé selon la revendication 23, **caractérisé en ce qu'**on applique le composé, qui contient dans la molécule au moins deux groupes, qui signifient, indépendamment l'un de l'autre, un groupe amino primaire, un groupe amino secondaire, un groupe carboxyle, un groupe amide, un groupe phosphono et/ou un groupe hydroxyle, ou le mélange de ces composés, à partir d'une solution aqueuse, le cas échéant acide ou basique, par mouillage ou par pulvérisation par le composé pur, sur la surface puis on chauffe le cas échéant à une température d'environ 80°C à 120°C, le cas échéant sous pression.

25. Procédé selon la revendication 24, **caractérisé en ce qu'**on met en contact le composé en solution aqueuse en une concentration d'au moins 10 µmoles/1 (micromoles par litre) avec la surface métallique hydroxylée.
